# DEMANDE DE BREVET EUROPEEN

(11) **EP 2 336 657 A1**
(43) Date de publication de la demande: **22.06.2011**
(21) Numéro de dépôt: 09392002.3
(22) Date de dépôt: 09.12.2009
(51) Int. Cl.: F24F 3/12, A61L 9/14

(54) **Procédé de dégraissage et de neutralisation des odeurs de conduites d'aération, et dispositif pour la mise en oeuvre du procédé**

(71) Demandeur: Lange, Alain, 98000 Monaco (MC)
(72) Inventeur: Lange, Alain, 98000 Monaco (MC)
(74) Mandataire: Schuffenecker, Thierry

(57) **Abrégé**

Un procédé et dispositif de dégraissage et de neutralisation des odeurs d'un ensemble de conduits de ventilation comportant les étapes:
- propulsion d'air au dessus d'au moins un transducteur(s) ultrasonique(s) apte(s) à émettre des ondes ultrasoniques susceptibles de pulvériser un produit actif sous forme liquide comportant des bactéries et/ou des enzymes susceptibles de biodégrader des graisses et des matières organiques, le ou les transducteur(s) ultrasonique(s) étant immergés à une profondeur h constante ;
- extraction de l'air propulsé vers un système de conduits de ventilation ou d'aération.

Plus particulièrement, le produit actif est stocké dans un premier réservoir inférieur et le ou les transducteurs ultrasonores sont disposés dans un second réservoir supérieur comportant un circuit de circulation continu de produit actif entre les deux réservoirs basé sur une pompe et une surverse assurant un niveau d'immersion constant du ou des transducteurs ultrasoniques.

L'invention est particulièrement adapté à la réalisation d'un dispositif de traitement d'une hotte de cuisine professionnelle.

## Description

### Domaine technique de l'invention

La présente invention concerne un procédé optimisé de dégraissage et de neutralisation des odeurs dans les conduits d'aération et de ventilation

### Etat de la technique

Il est connu un dispositif de diffusion d'un produit contenant des enzymes et des bactéries pouvant biodégrader les graisses et les matières organiques.

Pour être efficaces les goutelletes doivent être particulières réduites (quelques µm tout au plus) .

Une première méthode connue exige l'installation d'une pompe haute pression pour élever le mélange à une pression comprise entre 25 et 100 bars et l'installation de multples buses spécifiques qui, dans le meilleur des cas, formeront des gouttelettes dont la plus petite taille atteindra effectivement 10 µm).

Ce type d'installation nécessite de multples buses d'injection qui entraînent un coût élevé des installations et rend les opérations de contrôle et de maintenance fastidieuses et très onéreuses car les buses spécifiques doivent être remplacées plusieurs fois dans l'année d'autant plus que la plupart du temps les buses se trouvent éloignés dans des endroits peu accessibles tels que les faux plafonds ou gaines techniques.

Une second methode connue existante consiste à générer un brouillard par l'utilisation de têtes de micronisation fonctionnant à l'air comprimé. Mais cette méthode ne permet d'obtenir que de très faibles débits et nécessite une production d'air comprimé importante. Cette technique a également le désavantage d'être bruyante et de devoir faire fonctionner de manière continue des compresseurs ou pompes à air.

Par conséquent, il serait souhaitable de bénéficier d'une technique alternative permettant d'effectuer du dégraissage au moyen d'enzymes et de bactéries des conduits d'aération et de ventilation.

### Exposé de l'invention

La présente invention a pour but de proposer un procédé de dégraissage et de neutralisation des odeurs de conduits de ventilation, et d'un dispositif de mise en oeuvre du procédé.

La présente invention a pour but de proposer un procédé et un dispositif de diffusion d'un produit de dégraissage et de neutralisation des odeurs dans les conduits d'aérations de bâtiments professionnels ou à usage d'habitation.

Un autre but de la présente invention consiste à proposer un procédé et un dispositif de diffusion d'un produit destiné à la neutralisation des odeurs et/ou au dégraissage en continu de conduits de ventilation et d'extraction des vapeurs grasses des cuisines professionnels ou de tout autre conduit susceptible d'être encrassé par des graisses ou autres matières organiques.

L'invention réalise ces buts au moyen d'un procédé de dégraissage et de neutralisation des odeurs d'un ensemble de conduits de ventilation comportant les étapes:
- propulsion d'air au dessus d'au moins un transducteur(s) ultrasonique(s) apte(s) à émettre des ondes ultrasoniques susceptibles de pulvériser un produit actif sous forme liquide comportant des bactéries et/ou des enzymes susceptibles de biodégrader des graisses et des matières organiques, le ou les transducteur(s) ultrasonique(s) étant immergés à une profondeur h constante ;
- extraction de l'air propulsé vers un système de conduits de ventilation ou d'aération.

Plus particulièrement, le produit actif est stocké dans un premier réservoir inférieur et le ou les transducteurs ultrasonores sont disposés dans un second réservoir supérieur comportant un circuit de circulation continu de produit actif entre les deux réservoirs basé sur une pompe et une surverse assurant un niveau d'immersion constant du ou des transducteurs ultrasoniques.

De préférence, la déverse est réalisée au moyen d'un ou plusieurs tube(s) de surverse perçant le fond dudit second réserveur pour permettre l'écoulement du trop-plein dudit second réservoir dès lors que la hauteur du niveau dudit second réservoir atteind une valeur déterminée (h).

En particulier, le ou les tubes de surverse présente une longueur de 30 à 50 mm par rapport au fond dudit second réservoir.

Dans un mode de réalisation particulier, le procédé comporte la préparation, sur demande ou commande d'un unité de commande, d'une quantité de produit actif à partir d'un concentré stocké dans un troisième réservoir et d'eau fournie par une alimentation d'eau, et préalablement filtrée, ladite préparation étant automatiquement effectuée au sein d'un mélangeur préalablement au remplissage dudit premier réservoir.

L'invention réalise également un dispositif de dégraissage et de neutralisation des odeurs de conduits d'aération comportant :
- un carter comportant au moins un réservoir contenant du produit actif sous forme liquide comportant des bactéries et/ou des enzymes susceptibles de biodégrader des graisses et des matières organiques, ledit carter comportant au moins un conduit d'admission d'air, et au moins un conduit d'extraction pouvant être raccordé à un système de conduits de ventilation ou d'aération d'un immeuble industriel ou à usage d'habitation ;
- au moins un transducteur(s) ultra-sonique(s) immergé(s) dans ledit produit liquide, apte à émettre des ondes ultrasonores susceptibles de pulvériser ledit produit actif.

De préférence, le dispositif comporte :
- un premier réservoir inférieur destiné à recevoir un volume prédéfini de produit actif ;
- un second réservoir supérieur comportant le ou les transducteur(s) ultrasonique ;
- un circuit de circulation comportant une pompe de circulation pour générer un flux entre le premier et le second réservoir ;
- une surverse assurant le maintien du niveau de produit liquide dudit second réservoir à une hauteur constante (h) au dessus du ou des transducteurs.

Dans un mode de réalisation particulier, la surverse est réalisée au moyen d'un ou plusieurs tubes de services percant le fond dudit second réservoir supérieur pour verser le trop-plein dudit second réservoir dans ledit premier réservoir.

De preference, le ou les tubes de surverse présentent une longueur de 30 à 50 mm par rapport au fond dudit second réservoir.

Dans un mode de réalisation particulier, le dispositif se présente sous la forme d'une unité fonctionnelle comportant un volume prédéterminé de produit prêt à l'emploi dans ledit premier réservoir.

Alternativement, le dispositif se présente sous la forme d'une unite fonctionnelle comportant en outre des moyens pour préparer, sous contrôle d'une unité de commande, un volume prédéfini de produit actif destiné au remplissage dudit premier réservoir.

L'invention est particulièrement utile à la réalisation d'un procédé efficace de traitement des conduits d'aération en raison de l'efficacité du micro brouillard généré par les capteurs ultra soniques fonctionnant à une profondeur d'immeursion constante h déterminée par la hauteur du ou des tube(s) de surverse.

Plus particulièrement, l'invention s'avère adaptée à la realization d'un dispositif efficace de dégraissage et de neutralisation des odeurs d'une hotte de cuisine professionnelle, comportant :
- une unité fonctionnelle présentée sous la forme d'un carter comportant au moins un réservoir contenant du produit actif sous forme liquide comportant des bactéries et/ou des enzymes susceptibles de biodégrader des graisses et des matières organiques, ledit carter comportant au moins un conduit d'admission d'air (8), et au moins un conduit d'extraction (13) pouvant être raccordé à un système de conduits de ventilation ou d'aération d'un immeuble industriel ou à usage d'habitation ;
- au moins un transducteur(s) ultra-sonique(s) immergé(s) dans ledit produit liquide, apte à émettre des ondes ultrasonores susceptibles de pulvériser ledit produit actif.
- un tube raccordé à une extrémité au conduit d'extraction et disposant d'une seconde extrémité venant traverser une hotte de cuisine professionnelle, la seconde extrémité étant dotée d'un ensemble d'orifice apte à la diffusion du mélange air/brouillard généré par lesdits transducteurs ultrasoniques.

### Description des dessins

D'autres caractéristiques, but et avantages de l'invention apparaîtront à la lecture de la description et des dessins ci-après, donnés uniquement à titre d'exemples non limitatifs. Sur les dessins annexés :
La figure 1 illustre un premier mode de réalisation d'une unité de diffusion de produit destiné à la neutralisation des odeurs et/ou au dégraissage en continu de conduits de ventilation conforme à la présente invention.
La figure 2 illustre un second mode de réalisation d'une unité de diffusion de produit destiné à la neutralisation des odeurs et/ou au dégraissage conforme à la présente invention.
La figure 3 illustre un troisième mode de réalisation permettant la préparation automatisée, sur site, du produit actif destiné à la neutralisation des odeurs et/ou au dégraissage conforme à la présente invention.

### Description d'un mode de réalisation préféré

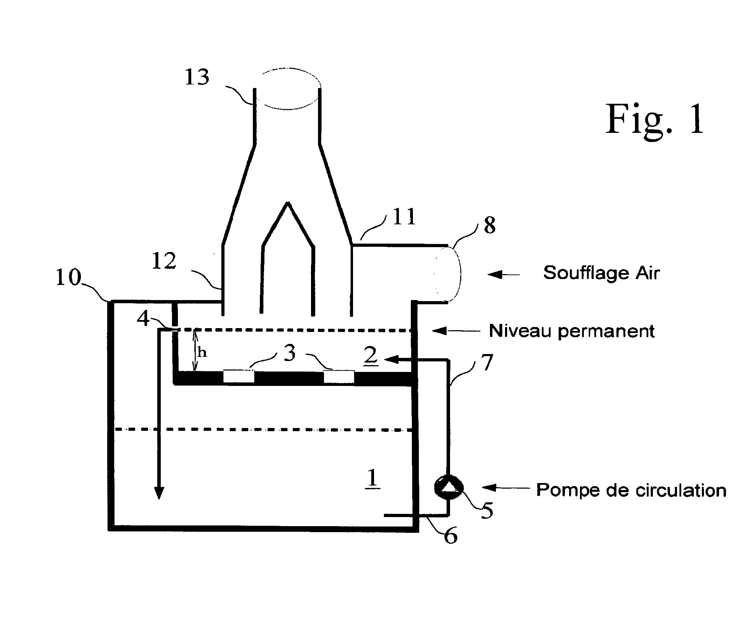

En référence à la figure 1 on décrit à présent un premier mode de réalisation d'un dispositif conforme à la présente invention et assurant la diffusion d'un produit actif pour le dégraissage et de neutralisation des odeurs dans les conduits de ventilation.

Ce premier mode de réalisation se présente sous la forme d'une unité autonome comportant un carter 10 comportant un double réservoir, à savoir une première cuve cuve inférieure 1 destinée au stockage d'un volume prédéterminé de produit actif présenté sous forme liquide et une seconde cuve supérieure 2 située au-dessus de la cuve 1 dotée, dans l'exemple illustré sur la figure 1, d'un conduit d'admission d'air 8 et de deux cheminées ou conduits d'extraction 11 et 12 localisés au-dessus de deux transducteurs ultrasoniques 3.

Si la figure 1 montre un exemple comportant deux transducteurs ultrasoniques, un homme du métier pourra clairement disposer un nombre plus important de transducteurs, le cas échéant .

D'une manière générale, dans ce premier mode de réalisation, la cuve 1 est dotée d'un conduit adéquat (non représenté dans la figure) permettant à tout le moins le remplissage périodique de cette cuve par un opérateur.

Une pompe de circulation 5, communiquant avec la cuve 1 via un conduit inférieur 6 et communiquant avec la cuve 2 via un conduit supérieur 7, assure une circulation en continu de produit liquide actif de la première cuve inférieure 1 vers la cuve supérieure 2.

D'une manière générale, le réservoir 1 pourra être doté d'un ou plusieurs capteur(s) de niveau permettant, d'une part, un fonctionnement optimal de la pompe de circultation 5 et, d'autre part, de fournir des signaux de détection adéquats pour alerter un opérateur ou une unité de commande adéquate (non illustrée) d'un niveau de liquide anormalement bas au sein de la cuve 1.

La cuve supérieure 2 comporte un élément 4, tel qu'un orifice assurant une fonction de surverse dans la première cuve inférieure 1 dès lors que le niveau de liquide atteint une hauteur h prédéfinie au-dessus du ou des transducteur(s) 3.

La pompe 5 assure ainsi une circulation permanente de produit liquide de la cuve 1 vers la cuve 2 de manière à maintenir constante le niveau de produit actif au-dessus des transducteurs ultrasoniques 3.

Chaque transducteur ultrasonique est associé à un circuit électronique (non représenté) permettant son activation dans le but de générer un brouillard extrèmement fin de produit actif, sous la forme de goutelettes extrèmement fines de l'ordre de 5 µm, pouvant être extraites de la cuve supérieure 2 par les conduits d'extraction 11 et 12 pour être acheminées, via le conduit d'extraction, vers les conduits de ventilation à traiter.

Le ou les transducteur(s) ultrasonique(s) 3 peuvent être des transducteurs conventionnelles permettant la génération d'un brouillard à partir de l'eau liquide à partir d'ondes ultrasoniques. D'une manière particulière, on pourra venir sélectionner le choix d'un transducteur et sa fréquence de fonctionnement en fonction de la nature particulière de la composition de produit actif. Clairement, un homme du métier pourra rechercher dans la gamme de fréquence couramment utilisée pour la vaporisation de liquide la fréquence la plus adaptée à une composition particulière de produits actifs.

Dans un mode de réalisation particulier, l'unité de fonctionnement comporte une unité de commande (non représentée) permettant le réglage de la fréquence de fonctionnement du ou des transducteur(s) 3.

Le mode de réalisation de la figure 1 réalise ainsi une unité fonctionnelle complète comportant un réservoir de produit actif, stocké dans la cuve inférieure 1, permettant de traiter pendant une durée prédéfinie les conduits de ventilations d'un bâtiment industriel ou à usage d'habitation.

On notera en particulier que même lorsque le niveau de produit tendra à baisser, du fait de la consommation constante de produit actif vaporisé par les transducteurs, le niveau de liquide reste constant dans la cuve supérieure assurant ainsi un fonctionnement optimal du ou des transducteur(s)

D'une part, ces transducteurs seront parfaitement refroidis puisqu'ils seront immergés dans un liquide présentant une hauteur suffisante (h).

D'autre part, ces mêmes transducteurs présenteront une performance optimale du fait que la hauteur de liquide ne tendra pas à varier. On assure ainsi un rendement constant du ou des transducteur et une bonne maîtrise de la régularité du volume de liquide actif pulvérisé alors même que le niveau de réservoir inférieur tendra à baisser au fur et à mesure de la consommation de produit actif.

On note même, et cela constitue un avantage considérable de la présente invention, qu'un opérateur pourra venir remplir le réservoir inférieur 1 alors même que le réservoir supérieur 2 est en fonctionnement, car les transducteurs ultrasoniques restents immergés à une hauteur optimale h. Il n'est par conséquent par nécessaire de devoir interrompre le fonctionnement de l'appareil ce qui est extrèmement avantageux car l'on sait que le traitement des odeurs dans un conduit d'aération ― notamment pour une installation professionnelle comme une cuisine professionnelle ― ne peut souffrir la moindre interruption.

Ainsi, l'unité décrite dans la figure 1 apportent des avantages décisifs en permettant de combiner l'efficacité de la production de brouillards par onde ultrasonore et ce d'une manière continue alors mêmes que l'on procède au remplissage de son réservoir inférieur par du produit actif.

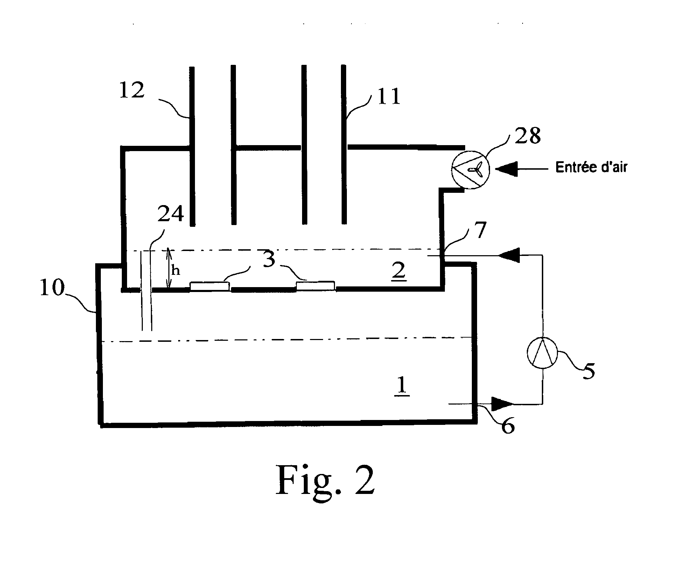

En référence à la figure 2 , on décrit à présent un second mode de réalisation d'une unité de diffusion d'un produit actif prêt à l'emploi pour le dégraissage et de neutralisation des odeurs dans les conduits de ventilation.

Dans un souci de simplification et de concision de l'exposé, les éléments qui sont communs avec ceux de la figure 1 comporteront les mêmes références numériques.

Comme on le voit, ce second mode de réalisation comporte une fois encore une première cuve inférieure 1 et une cuve supérieure 2, la cuve supérieure comportant un conduit d'admission ainsi qu'un ou plusieurs conduits d'extractions 11 et 12. Un ventilateur 28 placé sur la cuve supérieure sert à propulser l'air d'admission vers les conduits ou cheminées 11-12 en passant au-dessus des transducteurs ultra-sonores 3.

Le réservoir inférieur 1 comporte à nouveau la pompe de circulation 5 assurant la circulation en continu de produit actif du réservoir inférieur 1 vers le réservoir supérieur 2 via les conduits 6 et 7 .

L'on constate à présent que, dans ce second mode de réalisation, le trop-plein étant à présent assuré par au moins un tube de surverse 24 transperçant le fond de la cuve supérieure 2 et dont l'extrémité supérieure est située à une hauteur h par rapport au niveau de ce même fond.

Ainsi, le trop plein de produit actif apporté par la pompe de circulation 5 et les deux conduits 6 et 7, peut directement sécouler, via le ou les tubes de surverse 24, du réservoir 2 dans le réservoir inférieur 1.

On réalise ainsi, une fois encore comme dans le mode de réalisation de la figure 1, la fonction de surverse au sein du réservoir 2 de manière à maintenir constante la hauteur de produit actif au-dessus des transducteurs 2 et 3 .

Ce second mode de réalisation présente l'avantage, par rapport au premier mode de réalisation décrit en relation avec la figure 1, que l'on pourra faire varier la hauteur de liquide au ― dessus du ou des transducteur(s) 3 au moyen d'un ou plusieurs tube(s) de surverse de hauteur calibrée, choisie en fonction de la nature du produit actif à vaporiser. On pourra ainsi venir assurer un élément de réglage sur la production de brouillard actif en fonction de la nature du produit de traitement.

L'on pourra ainsi, au moyens de tube de surverse de hauteurs variables, venir régler précisément la profondeur d'immersion des transducteurs ultrasoniques 3.

Dans un mode de réalisation particulier, les tubes de surverse présentent une hauteur h au dessus des transducteurs ultrasoniques 3 comprise entre 30 et 50 mm.

Les premier et second modes de réalisation correspondent à une unité fonctionnelle indépendante permettant le traitement en continu de conduites de ventilation (c'est-à-dire sans nécessaire l'arrêt des transducteurs 3), et cela même lorsque un opérateur procède au remplissage manuel du réservoir inférieur 1 avec du produit prêt à l'emploi.

D'une manière générale, on pourra donner des formes concrètes diverses à l'unité fonctionnelle, et notamment prévoir un carter démontable en deux compartiments permettant d'accéder simplement aux deux réservoirs inférieur et supérieur pour les besoins du nettoyage et/ou de la maintenance.

Dans un mode de réalisation particulier, le fond du second réservoir comportant les transducteurs 3 pourra être prévu amovible et doté de connecteurs électriques adéquats (non représentés sur la figure) pour permettre un remplacement aisé de ces éléments lorsque ceux-ci seront encastrés. L'échange standard de ces éléments pourra être effectué sans difficulté.

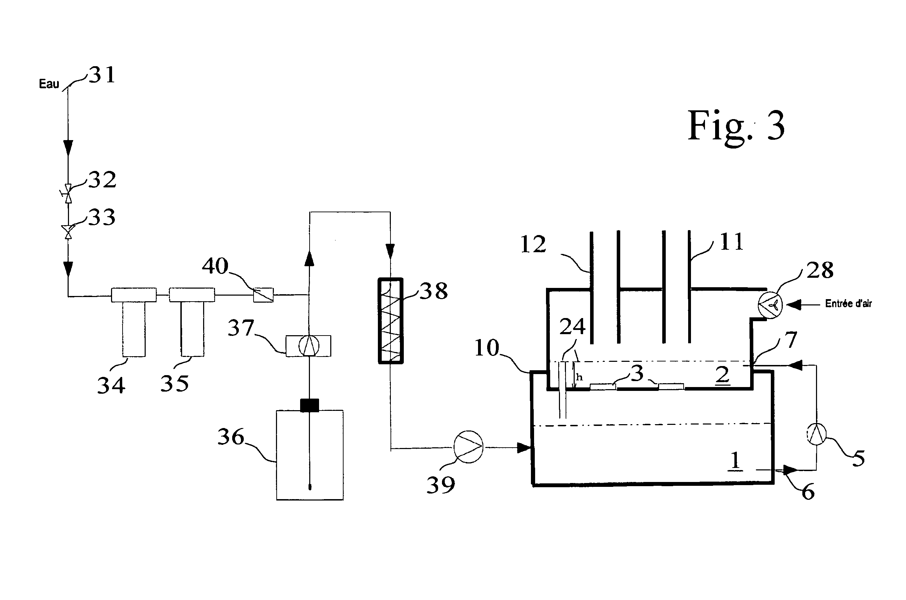

L'on décrit à présent en relation avec la figure 3, pour illustrer les nombreuses adaptations possibles de l'invention, un troisième mode de réalisation qui permet la préparation d'un mélange de produit actif en continu de manière à réaliser une unique complètement autonome, fabriquant son propre produit actif et ne nécessitant qu'un entretien épisodique.

Dans un souci de concision, les éléments communs avec ceux du second dispositif portent la même référence. Le troisième mode de réalisation comporte ainsi le réservoir inférieur 1, le réservoir supérieur 2, le conduit d'admission doté de son ventilateur 28, et les cheminées 11 et 12 situées en surplomb au-dessus du ou des transducteur(s) 3. Le troisième mode de réalisation comporte également le circuit de circulation se composant de la pompe de circulation 5, des conduits 6 et 7 ainsi que d'un ou plusieurs tube(s) de surverse 24.

Le troisième mode de réalisation comporte en outre des éléments complémentaires permettant une préparation sur demande de produit actif à partir d'un concentré stocké dans un réservoir 36 et d'eau alimentée par une arrivée d'eau 31.

D'amont en aval, l'arrivée d'eau 31 alimente en série une électrovanne électrovanne 32 commandée par une unité centrale (non représentée), un détendeur 33 destiné à réduire la pression d'entrée avant la préparation du mélange actif.

Le dispositif comporte, en sortie du détendeur 33, un dispositif de filtrage efficace, doté par exemple de deux filtres à eau 34 et 35 en cascade, assurant un filtrage efficace préalablement à la constitution du mélange. En aval du filtre 35, un clapet anti-retour assurer le sens unidirectionnel du fluide d'alimentation en eau.

Le dispositif comporte en outre une pompe de dosage 37 puisant dans le troisième réservoir 36 et permettant l'extraction d'une dose prédéfinie de concentré actif.

Un mélangeur 38 présente un conduit d'entrée connectée à la sortie du clapet anti-retour 40 ainsi qu'à la sortie de la pompe de dosage 37 de manière à permettre un mélange convenablement dosé produit actif 38 à partir du concentré contenu dans le réservoir 36 et l'eau filtrée arrivant du clapet anti-retour 40.

Le dispositif comporte enfin, en sortie du mélangeur 38, une pompe de remplissage 39 dont la sortie est connectée au réservoir inférieur 1 pour injecter, sur commande d'une unité centrale, le produit actif préparé dans le mélangeur 38 directement dans le réservoir inférieur 1 pour venir compenser, en tant que de besoin, le niveau de ce denier.

Il est clair que ce troisième mode de réalisation autorise une plus grande autonomie de fonctionnement puisque le produit actif est préparé et renouvelé en tant que de besoin.

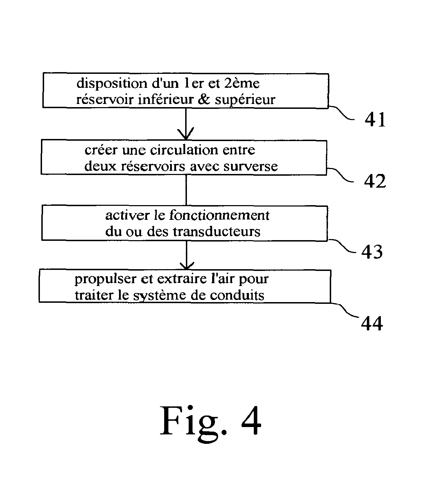

La figure 4 illustre plus particulièrement les différentes étapes mises en oeuvre dans le procédé selon l'invention, dont on a vu, avec les trois modes de réalisation, qu'il peut être mise en oeuvre sous des formes concrètes diverses.

Dans une étape 41, le procédé comporte, en premier lieu, la disposition d'au moins un premier réservoir inférieur et un second réservoir supérieur contenant respectivement un volume prédéterminé de produit actif sous forme liquide et au moins un transducteur(s) ultrasonique(s)

Dans une étape 42, le procédé comporte la mise en oeuvre d'une circulation de liquide actif entre ledit premier et second réservoir au moyen d'une pompe de circulation associée à une surverse de manière à maintenir constante la hauteur d'immersion du ou des transducteur(s) ultrasonique(s).

Dans une étape 43, le procédé comporte l'activation du ou des transducteurs en même temps que celle du circuit de circultation .

Dans une étape 44, le procédé comporte la propulsion d'air à l'intérieur du second réservoir et son extraction pour l'acheminer vers un système de conduits de ventilation et d'aération devant être dégraissés.

L'invention qui vient d'être décrite permet la réalisation d'une unité fonctionnelle extrêmement performante permettant l'application, d'une manière totalement nouvelle et avantageuse, toute une gamme de produits actifs prêt à l'emploi comportant des complexes d'huiles essentielles d'enzymes et de bactéries apte au dégraissage et qui, d'habitude sont appliqués au moyen de pulvérisateur conventionnels. On citera, à titre d'exemple nullement limitatif, le produit désigné par Bio-C du fabricant VAPORTEK (marque déposée).

L'invention permet, d'une manière particulièrement avantageuse, d'appliquer ces produits conventionnels dans les conduits de ventilation et d'aération qui, jusqu'à présent, ne permettaient pas leur application.

Dans un mode de réalisation tout à fait particulier, l'invention s'avère particulièrement adaptée à l'application produits actifs évoqués ci-dessus dans un endroit particulièrement difficile d'accès et réticent à une application par pulvérisation de ce type de produit, à savoir les hottes de cuisine professionnelles et autres capteurs.

A cet effet, l'on adjoint à l'une des unité fonctionnelles décrites précédemment, un tube, par exemple en acier inoxidable d'un diamètre de l'ordre de 40 mm, présentant une première extrémité raccordé au conduit d'extraction 13 et une seconde extrémité allant traverser de part une hotte de cuisine industrielle ou professionnelle, dans sa partie haute et sur une largeur prédéfinie.

La seconde extrémité est doté en outre d'un jeu d'orifices, par exemple de 10 mm de diamètre environ, espacé sur toute une longueur du tube correspondant à la largeur prédéfinie de la hotte. Dans un mode de réalisation particulier, on pourra espacer les orifices de 10 cm environ.

De cette manière, l'on permet une diffusion directement au centre de la hotte du mélance actif air/brouillard actif généré au sein de l'unité fonctionnelle et acheminé par le tube au coeur de la zone à traiter.

On a constaté que l'on pouvait ainsi procéder à un dégraissage efficace de la hotte de cuisine et neutraliser les odeurs dans un endroit particulièrement sensible qui, une fois encore, ne peut souffrir la moindre interrupition de traitement.

## Revendications

1. Procédé de dégraissage et de neutralisation des odeurs d'un ensemble de conduits de ventilation comportant les étapes:
- propulsion d'air au dessus d'au moins un transducteur(s) ultrasonique(s) apte(s) à émettre des ondes ultrasonores susceptibles de pulvériser un produit actif sous forme liquide comportant des bactéries et/ou des enzymes susceptibles de biodégrader des graisses et des matières organiques, le ou les transducteur(s) ultrasonique étant immergés à une profondeur h constante ;
- extraction de l'air propulsé vers un système de conduits de ventilation ou d'aération.

2. Procédé selon la revendication 1 **caractérisé en ce qu'**il comporte les étapes de :
- disposer (41) au moins un premier et un second réservoir respectivement inférieur et supérieur comportant respectivement un volume de produit actif et le ou les transducteur(s) ultrasonique(s) ;
- effectuer (42) une circulation de produits en continu entre lesdits premiers et second réservoirs au moyen d'une ensemble pompe et surverse;
- activer ladite circulation durant le fonctionnement des capteurs ultrasoniques.

3. Procédé selon la revendication 2 **caractérisé en ce que** le maintien constant dudit niveau est réalisé au moyen d'un ou plusieurs tube(s) de surverse perçant le fond dudit second réserveur pour permettre l'écoulement du trop-plein dudit second réservoir dès lors que la hauteur du niveau dudit second réservoir atteind une valeur déterminée (h).

4. Procédé selon la revendication 3 **caractérisé en ce que** le ou les tubes de surverse présente une longueur de 30 à 50 mm par rapport au fond dudit second réservoir.

5. Procédé selon la revendication 2 **caractérisé en ce qu'**il comporte les étapes suivantes :
- préparation, sur demande, d'une quantité de produit actif à partir d'un concentré stocké dans un troisième réservoir et d'eau fournie par une alimentation d'eau, et préalablement filtrée, ladite préparation étant automatiquement effectuée au sein d'un mélangeur préalablement au remplissage dudit premier réservoir.

6. Procédé selon la revendication 3 **caractérisé en ce qu'**il comporte la mise en oeuvre d'une pompe de dosage destiné au remplissage dudit mélangeur.

7. Dispositif de dégraissage et de neutralisation des odeurs de conduits d'aération comportant :
- un carter comportant au moins un réservoir contenant du produit actif sous forme liquide comportant des bactéries et/ou des enzymes susceptibles de biodégrader des graisses et des matières organiques, ledit carter comportant au moins un conduit d'admission d'air (8), et au moins un conduit d'extraction (13) pouvant être raccordé à un système de conduits de ventilation ou d'aération d'un immeuble industriel ou à usage d'habitation ;
- au moins un transducteur(s) ultra-sonique(s) immergé(s) dans ledit produit liquide, apte à émettre des ondes ultrasonores susceptibles de pulvériser ledit produit actif.

8. Dispositif selon la revendication 7 **caractérisé en ce qu'**il comporte :
- un premier réservoir inférieur (1) destiné à recevoir un volume prédéfini de produit actif ;
- un second réservoir supérieur (2) comportant le ou les transducteur(s) ultrasonique ;
- un circuit de circulation comportant une pompe de circulation pour générer un flux entre ledit premier (1) et le second réservoir (2) ;
- une surverse (4) assurant le maintien du niveau de produit liquide dudit second réservoir à une hauteur constante (h) au dessus du ou des transducteurs.

9. Dispositif selon la revendication 8 **caractérisé en ce que** la surverse (4) est réalisée au moyen d'un ou plusieurs tubes de services percant le fond dudit second réservoir supérieur pour verser le trop-plein dudit second réservoir dans ledit premier réservoir.

10. Dispositif selon l'une des revendication 9 **caractérisé en ce que** le ou les tubes de surverse présente(nt) une longueur de 30 à 50 mm par rapport au fond dudit second réservoir.

11. Dispositif selon l'une des revendication 7 ou 10 **caractérisé en ce qu'**il consiste en une unité fonctionnelle comportant un volume prédéterminé de produit prêt à l'emploi dans ledit premier réservoir.

12. Dispositif selon l'une des revendications 7 à 10, **caractérisé en ce qu'**il comporte en outre des moyens (31-40) pour préparer, sous contrôle d'une unité de commande, un volume prédéfini de produit actif destiné au remplissage dudit premier réservoir (1).

13. Dispositif selon la revendication 12 **caractérisé en ce qu'**il comporte :
- une arrivée d'eau (31) ;
- une électrovanne (32) commandée par ladite unité de commande;
- un détendeur (33) ;
- des moyens de filtration (34, 35) ;
- un clapet antiretour (40) ;
- un troisième réservoir de stockage de concentré actif (36) ;
- une pompe de dosage destinée à alimenter un mélangeur (38) d'une dose prédéfinie de concentré actif stocké dans ledit troisième réservoir (36) ;
- un mélangeur (38) destiné à recevoir une composition prédéterminé d'eau filtrée et de concentré actif dosé par ladite pompe de dosage ;
- une pompe de remplissage pour extraire la préparation dudit mélangeur et la conduire dans ledit premier réservoir (1)

14. Dispositif selon l'une quelconque des revendications 13 **caractérisé en ce que** le remplissage dudit premier réservoir est effectué alors que le ou les transducteurs ultrasoniques sont en fonctionnement dans ledit second réservoir.

15. Dispositif de dégraissage et de neutralisation des odeurs d'une hotte de cuisine professionnelle, comportant :
- une unité fonctionnelle présentée sous la forme d'un carter comportant au moins un réservoir contenant du produit actif sous forme liquide comportant des bactéries et/ou des enzymes susceptibles de biodégrader des graisses et des matières organiques, ledit carter comportant au moins un conduit d'admission d'air (8), et au moins un conduit d'extraction (13) pouvant être raccordé à un système de conduits de ventilation ou d'aération d'un immeuble industriel ou à usage d'habitation ;
- au moins un transducteur(s) ultra-sonique(s) immergé(s) dans ledit produit liquide, apte à émettre des ondes ultrasonores susceptibles de pulvériser ledit produit actif.
- un tube raccordé à une extrémité au conduit d'extraction et disposant d'une seconde extrémité venant traverser une hotte de cuisine professionnelle, la seconde extrémité étant dotée d'un ensemble d'orifice apte à la diffusion du mélange air/brouillard généré par lesdits transducteurs ultrasoniques.
